# EUROPÄISCHE PATENTANMELDUNG

(11) **EP 2 082 733 A1**
(43) Veröffentlichungstag der Anmeldung: **29.07.2009**
(21) Anmeldenummer: 09000085.2
(22) Anmeldetag: 07.01.2009
(51) Int. Cl.: A61K 9/06

(54) **Verwendung von Lipidkomponenten gegen Pollenallergien**

(30) Priorität: 28.01.2008 DE 102008006393
(71) Anmelder: Beiersdorf AG, 20245 Hamburg (DE)
(72) Erfinder: Kröpke, Rainer, 22869 Schenefeld (DE); Bohnsack, Kerstin, Dr., 22399 Hamburg (DE); Rippke, Frank, Dr., 22527 Hamburg (DE); Filbry, Alexander, Dr., 22459 Hamburg (DE)
(74) Vertreter: Wilke, Jochen

(57) **Zusammenfassung**

Verwendung von Lipiden oder Lipidgemischen zur Herstellung einer kosmetischen oder dermatologischen Zubereitung zur Bekämpfung oder Prophylaxe von Pollenallergien, **dadurch gekennzeichnet, daß** der Spreitwert der Lipidphase der erfinderischen Zubereitungen unter 800 mm²/10 Min. bei 25° C und die Viskosität der eingesetzten Lipide/Lipidphase im Bereich von 40-25000 mPas liegt.

## Beschreibung

Die Erfindung betrifft die Verwendung bestimmter Lipidkomponenten in kosmetischen oder dermatologischen Zubereitungen gegen Pollenallergien.

Pollen bestimmter Bäume, Gräser, Getreidesorten und Kräuter rufen bei den Betroffenen eine laufende oder verstopfte Nase, juckende Augen und einen ständigen Niesreiz hervor.

Der Pollen oder Blütenstaub wird in den Staubbeutel der Samenpflanzen gebildet. Er besteht aus den Pollenkörnern, den Mikrosporen. Die Pollenkörner sind von einer widerstandsfähigen Zellwand umgeben, die unter anderem aus Sporopollenin besteht.

Pollenkörner dienen dazu, die männlichen Sporen geschützt zu den weiblichen Empfangsorganen zu bringen und so die Bestäubung und in weiterer Folge die Befruchtung zu gewährleisten.

Allergische Reaktionen auf Pflanzenpollen sind in zunehmendem Maße ein Problem.

Allergien und Hypersensitivität können sich äußern:
- an den Schleimhäuten (allergische Rhinitis (Heuschnupfen), Mundschleimhautschwellungen, Konjunktivitis (Bindehautentzündung))
- an den Atemwegen (Asthma bronchiale)
- an der Haut (atopische Dermatitis (Neurodermitis), Kontaktekzem, Urtikaria)
- im Gastrointestinaltrakt (Erbrechen, Durchfälle, besonders bei Säuglingen und Kleinkindern)
- als akuter Notfall (anaphylaktischer Schock).

Allergiker können an einer Krankheitsform leiden, aber auch an Mischformen. Während allergische Symptome an den Schleimhäuten typischerweise eher akut auftreten, können Symptome wie Asthma bronchiale und atopische Dermatitis einen chronischen Verlauf nehmen.

Erste Anzeichen von Pollenallergie können bei Kindern bereits zwischen dem fünften und dem sechsten Lebensjahr auftreten. Heuschnupfen ist für Kinder und deren Eltern eine Plage. Die vom Heuschnupfen geplagten Kinder leiden unter juckenden und tränenden Augen, Schnupfen, Atembeschwerden und Müdigkeit. Weil zudem die Gefahr besteht, daß sich aus dem Heuschnupfen ein Bronchialasthma entwickelt, sind konsequenter Pollenschutz und vorbeugende Maßnahmen sehr wichtig.

Zur Linderung akuter Symptome können Antihistaminika und Kortison eingesetzt werden. Die neuen Heuschnupfenmittel mit einem Antihistaminikum als Wirkstoff wirken sehr zuverlässig und machen, im Gegensatz zu ihren Vorgängern, kaum noch müde. Trotzdem ist bei erwachsenen Personen beim Führen von Fahrzeugen oder beim Bedienen von Maschinen Vorsicht geboten.

Pollenflug kann ferner Neurodermitis auslösen oder verstärken. Allergene aus der Luft können nicht nur Asthma und Heuschnupfen, sondern auch Reaktionen an der Haut auslösen. Jüngste Untersuchungen mit einem an der Technischen Universität München (Prof. Johannes Ring) entwickelten Test hätten ergeben, dass Pollen von Birken, Hasel, Erlen, Gräsern und Kräutern auf der Haut bestimmter Menschen Ekzeme auslösten - und Neurodermitis ist letztlich ein Ekzem. Dabei seien von dieser Art der Pollenallergie an der Haut auch Menschen betroffen, die nicht unter Heuschnupfen oder anderen Allergien litten.
Dass bei Neurodermitis auch Allergien im Spiel sind, war lange umstritten - man ging von einer angeborenen Krankheit aus, die durch psychische Einflüsse verstärkt wird.

Überraschend wurde gefunden, daß die Verwendung von Lipiden oder Lipidgemischen zur Herstellung einer kosmetischen oder dermatologischen Zubereitung zur Bekämpfung oder Prophylaxe von Pollenallergien, dadurch gekennzeichnet, daß der Spreitwert der Lipidphase der erfinderischen Zubereitungen unter 800 mm²/10 Min. bei 25°C und die Viskosität der eingesetzten Lipide/Lipidphase im Bereich von 40-25000 mPas liegt, den Nachteilen des Standes der Technik abhilft.

Besonders bevorzugt liegt der Spreitwert der Lipidphase unter 600 mm²/10 Min. bei 25°C und ganz besonders bevorzugt unter 400 mm²/10 Min. bei 25°C.

Die Viskosität der eingesetzten Lipide und/oder Lipidphase muss im Bereich von 40-25000 mPas , bevorzugt über 80-15000 mPas und ganz besonders bevorzugt über 100-8000 mPas liegen.

Die Messung des Spreitwertes (in mm²/10 min) erfolgt nach folgendem Verfahren. 20 µl der zu untersuchenden Substanz werden auf ein Rotband-Filterpapier der Fa. Schleicher & Schüll mittig getropft. Sogleich wird eine Stoppuhr gestartet und nach 10 Minuten die Fläche gemessen, die in dieser Zeit von der Substanz benetzt wurde. Die Messung wird in einem temperaturkostanten Raum mit 25°C +/- 1 °C durchgeführt.

Solche Zubereitungen verhindern oder vermeiden bei den betroffenen Personen allergische Reaktionen auf Pollen und schaffen dem Patienten bzw. Anwender solcher Zubereitung auf dem Vorwege Linderung allergischer Reaktionen

| Bevorzugte Lipide sind : | | |
|---|---|---|
| Handelsname | **INCI-Name** | **Viskosität (in mPas)** **(s⁻¹ = 500)** |
| Avocadoöl raffiniert DAC | Persea Gratissima | 180 |
| Shell Ondina 4222 | Mineral Oil | 43,9 |
| Myritol 331 | Cocoglycerides | 41 |
| Crodamol PTIS 3797 | Pentaerythrityl Tetraisostearate | 390 |
| Pionier 6301 | Mineral Oil | 51 |
| Eutanol G | Octyldodecanol | 47,7 |
| Miglyol 829 | Caprylic/Capric/Diglyceryl Succinate | 117 |
| Cosmacol ETI | Di-C12/13 Alkyl Tartrate | 107 |
| Pionier 2071 | Mineral Oil | 98 |
| Silkflo 366 NF | Polydecene | 45 |
| Abil Wax 9840 | Cetyl Dimethicone | 171 |
| Jojoba Oil FP35 Golden | Buxus Chinensis | 150 |
| Uniphen P-23 | Phenoxyethanol, Methylparaben, | 54,3 |
| | Ethylparaben, Propylparaben, Butylpara- | |
| | ben, Isobutylparaben | |
| Fluilan | Lanolin Oil | 3240 |
| Parsol MCX | Ethylhexyl Methoxycinnamate | 81,2 |
| Macadamianussoel | Macadamia Ternifolia | 180 |
| Motif | Squalan | 220 |
| Lipovol MOS-130 | Tridecyl Stearate(+) Tridecyl Trimellitate(+) | 47 |
| | Dipentaerythrityl Hexacapry- | |
| | late/Hexacaprate | |
| Rewopal PIB 1000 | Polyisobuten | 22170 |
| Polysynlan | Hydrogenated Polyisobuten | 57 |
| Prisorine 2041 GTIS | Triisostearin | 99 |
| Castor Oil PH.EUR | Ricinus Communis | 820 |
| Wacker AK 100 | Dimethicone | 101 |
| Ucon Fluid AP | PPG-14 Butyl Ether | 96 |
| Crodamol BS | Butyl Stearate | 100 |
| Dermol 488 | PEG-2 Diethylhexanoate | 110 |
| Dermosoft MCA | Dipropylene Glycol + Caprylyl Glycol + | 65 |
| | Glyceryl Laurate + Phenylpropanol | |
| | Hydroxystearate Glycerol Triacetyle | 65 |
| DUB DIAMOND | C10-40 Isoalkyl Acid Triglycerides | 132 |
| DUB MDIS | Diisostearyl Malate | 113 |
| DUB OLIOSE | Cannabis Sativa Seed Oil | 46 |
| DUB PTIS | Pentaerythrityl Tetraisostearate | 57 |
| DUB TMI | Triisodecyl Trimelliate | 111 |
| DUB TMTD | Tridecyl Trimellitate | 116 |
| Emerest 2384 | Propylene Glycol Monoisostearate | 42 |
| Hostaphat B 310 | Tributoxyethylphosphate | 122 |
| Hydrobrite 1000 PO | Hydrogenated Petrolatum | 568 |
| Isodragol | Triisononanoin | 67 |
| Lipex Olive | Olive Oil | 78 |
| Mobil PureSyn 1000 | Hydrogenated C6-14 Olefin Polymers | 3450 |
| Trivent PE 48 | Pentaerythrityl Tetraethylhexanoate | 116 |
| Wacker Belsil 1000 | Dimethicone | 1177 |
| Wacker Belsil 2000 | Dimethicone | 2306 |
| Wacker Belsil 350 | Dimethicone | 480 |
| Wacker Belsil 500 | Dimethicone | 585 |
| Wacker Silikonöl AK 50 | Polydimethylsiloxan | 51 |

Die Viskosität der Lipidphase kann durch rheologische Modifizierer und Lipidverdicker erhöht werden. Bevorzugte Lipidverdicker sind Hectorite, Silicate ("Aerosil 972"), Metallsalze der Stearinsäure, hydriertes Rizinusöl, Mikrowachse mit einem Schmelzpunkt über 42°C, Paraffine mit einem Schmelzpunkt über 42°C, native Wachse mit einem Schmelzbereich von 33-78°C, Glycerylester der Isotearate, Füllstoffe mit einer spezifischen Oberfläche von mindestens 0,2 m²/g , langkettige Silikonöle und Bentonite und/oder modifizierte Bentonite.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 3-(4-Hydroxy-3-methoxyphenyl)-1-(4-hydroxyphenyl)-propan-1-on.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen.

Es ist auch erfindungsgemäß vorteilhaft, 3-(4-Hydroxy-3-methoxyphenyl)-1-(4-hydroxyphenyl)-propan-1-on in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, 3-(4-Hydroxy-3-methoxyphenyl)-1-(4-hydroxyphenyl)-propan-1-on in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Gelbildner, Schaumstabilisatoren, Elektrolyte, organische Lösemittel oder Siliconderivate

Vorteilhafte Gelbildner für derartige Zubereitungen sind beispielsweise Copolymere aus C10-30-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon)

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 980, 981, 984 1342, 1382, 2984 und 5984. ebenso die ETD-Typen 2001, 2020, 2050 und Carbopol Ultrez 10, PVM/MA Decadien Crosspolymer (Handelsname Stabileze® 06), Polyglycerylmethacrylat sowie Polyacrylamid, Ammoniumdimethyltauramide/vinylformamidcopolymere, Copolymere oder Crosspolymere umfassend Aacryloyldimethyltaurate, Polyacryloyl-dimethyltauramide, Polyvinylpyrrolidone und Copolymere davon.

Weitere vorteilhafte Gelbildner für derartige Zubereitungen sind Xanthan Gummi, Polyvinylpyrrolidon, Cellulosederivate, insbesondere Celluloseether wie zum Beispiel Hydroxypropylmethylcellulose, Stärke und Stärkederivate, Hyaluronsäure, Carrageenan, Siliciumdioxid und Aluminiumsilikate.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethicone, Cyclomethicone, Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhaft können erfindungsgemäße Zubereitungen außerdem Substanzen enthalten, die als Stabilisator wirken, wobei die Gesamtmenge dieser Stabilisatoren z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die langzeitstabile Zubereitungen ermöglichen. Bevorzugte Stabilisatoren sind, Substanzen aus der Gruppe der Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3 , Sarcosin, Saxifraga Sarmentosa Extrakt, Scutellaria Baicalensis Extrakt , Natriummetabisulfit, Sojaisoflavon, Tocopherylglucosid, Trideceth-6, Zinkgluconat.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| **Beispiel Nr.** | **1** | **2** | **3** |
|---|---|---|---|
| Zyklisches Silikonöl | | | 3 |
| Sorbitanstearate | | | 2 |
| Lanolinalkohol | 3 | | |
| Polyglyceryl-2 Dipolyhydroxystearat | | 3 | |
| Polyglyceryl-3 Diisostearat | | 2 | |
| Cetylpalmitat | | | 10 |
| Vitamin E Acetat | | 1 | 0,5 |
| Propylparaben | | 0,15 | 0,1 |
| Methylparaben | | 0,4 | 0,3 |
| Mikrowachs | 15 | | |
| Mikrowachs + medizinisches Weißöl | 41 | | |
| Medizinisches Weißöl | 38,75 | 10 | 6 |
| Medizinisches Weißöl, dünnflüssig | | | 2 |
| Glycerin | 1 | 8,7 | 10 |
| Cetyalkohol | | | 3 |
| Aluminiumsalz von Stärkeoctenyl-bernsteinsäure | | | 1,5 |
| Isopropyl Stearate | | 11,25 | |
| Parfum | | 0,15 | 0,15 |
| Zitronensäure | | 0,086 | 0,086 |
| Natriumcitrat | | 0,174 | 0,174 |
| Magnesiumsulfat | | 0,6 | |
| Bisabolol | 0,25 | | |
| Carbomer, Natrium-Salz | | | 0,28 |
| Phenoxyethanol | | 0,8 | 0,8 |
| Cetearyl Alcohol | | | |
| Cetyl Alcohol | | | |
| Cetyl Palmitate | | | |
| PEG-150 Distearate | | | |
| Panthenol | 1 | 4 | 6,7 |
| Wasser | | ad 100 | ad 100 |
| | | | |
| pH - Wert | | 5 +/-0.7 | 5 +/-0,7 |

## Patentansprüche

1. Verwendung von Lipiden oder Lipidgemischen zur Herstellung einer kosmetischen oder dermatologischen Zubereitung zur Bekämpfung oder Prophylaxe von Pollenallergien, **dadurch gekennzeichnet, daß** der Spreitwert der Lipidphase der erfinderischen Zubereitungen unter 800 mm²/10 Min. bei 25° C und die Viskosität der eingesetzten Lipide/Lipidphase im Bereich von 40-25000 mPas liegt.

2. Verwendung nach Patentanspruch 1 **dadurch gekennzeichnet, daß** der Spreitwert der Lipidphase unter 600 mm²/10 Min. bei 25 °C und ganz besonders bevorzugt unter 400 mm²/10 Min. bei 25 °C.liegt.

3. Verwendung nach einem der vorangehenden Patentansprüche **dadurch gekennzeichnet, daß** die Viskosität der eingesetzten Lipide/Lipidphase im Bereich von 40-25000 mPas , bevorzugt über 80-15000 mPas und ganz besonders bevorzugt über 100-8000 mPas liegt.

4. Verwendung nach einem der vorangehenden Patentansprüche **dadurch gekennzeichnet, dass** Stabilisatoren enthalten sind.

5. Verwendung nach einem der vorangehenden Patentansprüche **dadurch gekennzeichnet, dass** die Stabilisatoren aus der Gruppe der Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3 , Sarcosin, Saxifraga Sarmentosa Extrakt, Scutellaria Baicalensis Extrakt , Natriummetabisulfit, Sojaisoflavon, Tocopherylglucosid, Trideceth-6, Zinkgluconat gewählt werden.

6. Verwendung nach einem der vorangehenden Patentansprüche **dadurch gekennzeichnet, daß** die Lipide der Lipidphase gewählt werden aus der Gruppe der medizinischen Weißöle, der Alkylester von C8-C30 Fettsäuren und der verzweigten Ester der C8-C24 Alkohole und der linearen Silikonöle.
